# EUROPÄISCHE PATENTSCHRIFT

(11) **EP 2 167 456 B1**
(45) Veröffentlichungstag und Bekanntmachung des Hinweises auf die Patenterteilung: **07.12.2011**
(21) Anmeldenummer: 08760734.7
(22) Anmeldetag: 09.06.2008
(51) Int. Cl.: C07C 209/78, C01B 7/05, C01B 7/07, B01D 15/00, B01J 20/18

(54) **Verfahren zur Rückgewinnung von Mineralsäuren bei der Herstellung von Diphenylmethandiamin**
Method for the recovery of mineral acids in a process for producing diphenylmethane diamine
Procédé de récupération d'acides minéraux dans un procédé de préparation de diphénylméthanediamine

(30) Priorität: 12.06.2007 EP 07110073
(43) Veröffentlichungstag der Anmeldung: 31.03.2010
(73) Patentinhaber: BASF SE, 67056 Ludwigshafen (DE)
(72) Erfinder: WLOKA, Veronika, 68163 Mannheim (DE); MATTKE, Torsten, 67251 Freinsheim (DE); KNÖSCHE, Carsten, 67150 Niederkirchen (DE); BREUNINGER, Daniel, 67240 Bobenheim-Roxheim (DE); BOTTKE, Nils, 68165 Mannheim (DE)
(86) Internationale Anmeldenummer: PCT/EP2008/057170
(87) Internationale Veröffentlichungsnummer: WO 2008/152021

(56) Entgegenhaltungen:
- WO-A-2005/007613
- DE-A1- 10 116 316
- EUGEN MÜLLER ED - HOUBEN-WEYL (HERAUSGEBER E MULLER): "METHODEN DER ORGANISCHEN CHEMIE, Band I/1" ALLGEMEINE LABORATORIUMSPRAXIS; [METHODEN DER ORGANISCHEN CHEMIE], STUTTGART, G. THIEME VERLAG, DE, Bd. BAND 01, 1. Januar 1958 (1958-01-01), Seiten 557-558, XP002306225

## Beschreibung

Der Begriff Mineralsäuren ist in der Technik zumeist eine Sammelbezeichnung für die drei starken anorganischen Säuren Salzsäure, Salpetersäure und Schwefelsäure. Gelegentlich wird auch Phosphorsäure zu dieser Stoffgruppe gezählt.

Mineralsäuren fallen in vielen organischen und anorganischen Prozessen als Nebenprodukte an oder werden als Katalysatoren (Säurekatalysatoren) eingesetzt.

Chlorwasserstoff entsteht beispielsweise in großer Menge bei der Chlorierung von Kohlenwasserstoffen, der Synthese von Chlorfluormethanen aus Tetrachlormethan, sowie der Phosgenierung von Aminen zu Isocyanaten.

Schwefelsäure wird als Nebenprodukt bei Sulfoxidationen erhalten.

Als homogener Katalysator findet Salzsäure beispielsweise Anwendung bei der Herstellung von Diphenylmethandiamin (MDA) aus Anilin und Formaldehyd.

Schwefelsäure wird beispielsweise bei der Synthese von Ethylenglykol aus Ethylenoxid oder bei der Dehydratisierung von Diethanolamin zu Morpholin als Katalysator eingesetzt. Ein weiterer technisch wichtiger Einsatzfall der Schwefelsäure ist die Herstellung von ε-Caprolactam aus Cyclohexanonoxim durch Umlagerung mit Schwefelsäure.

In vielen Synthesen mit Beteiligung von Mineralsäuren werden die Säuren im Anschluss an die Reaktion mit Laugen neutralisiert. Beispielsweise werden in der o. g. Chlorfluormethan-Synthese nach Abtrennung eines Großteils von Chlorwasserstoff die verbleibenden Reste in einer Laugenwäsche ausgewaschen.

Bei der Synthese von ε-Caprolactam wird durch Zugabe von Ammoniak das zwischenzeitlich gebildete Lactamsulfat zu freiem Lactam unter Freisetzung von Ammoniumsulfat umgesetzt.

Bei der MDA-Synthese wird durch Zugabe einer Lauge zum Reaktionsgemisch die als Katalysator wirkende Salzsäure neutralisiert und damit eine Phasentrennung zwischen organischer Wertproduktphase und chloridhaltiger wässriger Phase erreicht. Die entstehende Salzlösung wird zumeist entsorgt. Zur Behebung dieses Mangels wurde beispielsweise in WO 2005/007613 vorgeschlagen, auf eine Neutralisation zu verzichten und die Säure mit einem Adsorptionsmittel aus der Reaktionsmischung zu entfernen. Nachteilig ist hierbei jedoch, dass es zu keiner Phasentrennung kommt und die Adsorptionsmittel durch die organischen Amine belegt werden. Dadurch wird die Abtrennung der Säure beeinträchtigt.

In vielen Fällen werden die nach der Neutralisation bzw. Wäsche erhaltenen Salzlösungen als Abwasser entsorgt. Damit entstehen neben den Kosten für Säure und Lauge weitere Kosten für die Abwasserentsorgung.

Es war die Aufgabe der vorliegenden Erfindung, ein Verfahren zur Rückgewinnung von Säuren und Basen aus neutralisierten wässrigen Lösungen zu entwickeln. Dabei sollte die Säure möglichst frei von organischen Verunreinigungen sein, so dass sie im Verfahren wieder eingesetzt oder als Wertstoff verkauft werden kann.

Die Aufgabe konnte überraschenderweise dadurch gelöst werden, dass man den sauren Katalysator beziehungsweise die bei der Reaktion entstehende Säure nach Neutralisation mit einer basischen Verbindung und Separation der Salzlösung vom Wertprodukt über einen sauren Adsorber führt.

Gegenstand der Erfindung ist demzufolge ein Verfahren zur Gewinnung von Säuren aus Produktströmen bei der Herstellung von Diphenylmethandiamin, umfassend die Schritte
a) Umsetzung von Anilin mit Formaldehyd in Gegenwart einer Mineralsäure,
b) Neutralisation der Mineralsäure mit einer Base,
c) Trennung der organischen und der anorganischen Phase,
d) Entfernung der Base aus der anorganischen Phase mittels Adsorption,
e) Gewinnung der Mineralsäure.

Auf dem Adsorber wird die Base zurückgehalten während die Säure den Adsorber passiert. In einem Regenerierungsschritt kann die adsorbierte Base freigesetzt werden. Die auf diese Weise freigesetzten Säuren bzw. Basen können entweder einer weiteren Verwendung zugeführt oder aber in den Prozess zurückgeführt werden.

Die Adsorber sind vorzugsweise saurer Natur und/oder können Komplexe mit der Base bilden. Bevorzugt sind natürliche oder künstliche Silikate, besonders bevorzugt Zeolithe. Die Adsorber können gegebenenfalls dotiert werden. Zur Adsorption geeignet sind weiterhin metallhaltige (mit Ni, Cu, Cr oder anderen Komplexbildnern) dotierte Zeolithe. An den aktiven Zentren wird die Base gebunden, während die Säure den Adsorber passiert.

Als Adsorber können vorzugsweise saure Ionenaustauscher in der H-Form eingesetzt werden, wobei Zeolithe besonders bevorzugt sind.

Als Zeolithe werden insbesondere solche mit einer Struktur, die ausgewählt ist aus HEU, BEA, MOR, CHA, FAU, MFI, MEL, MWW, MTW, ZBM-11, FER, LTL, MAZ oder MCM-41, eingesetzt Die Abkürzungen sind für den Fachmann allgemein bekannt und beispielsweise im Atlas of Zeolite Framework Typesoder in der Database of Zeolite Structures von der International Zeolite Association veröffentlicht.

Bevorzugt werden die als Adsorber verwendeten Zeolithe in der H-Form eingesetzt.

In einer Ausführungsform des erfindungsgemäßen Verfahrens werden als Adsorber Zeolithe in der H-Form eingesetzt, die mit Metallen dotiert sind, wie Nickel, Chrom, Kupfer, Silber oder Kobalt. Die Menge der dotierten Metalle beträgt 0,5-10 Gew-%, bevorzugt 1-6 Gew-%, bezogen auf das Gewciht des dotierten Zeoliths.

Die erfindungsgemäß verwendeten Adsorber werden bevorzugt in Form von Kugeln, Stränglingen, Hohlzylindern, Ringen, Tabletten oder Split eingesetzt. Das Verhältnis aus äußerer Oberfläche und Volumen der Formkörper beträgt dabei > 0,2 mm⁻¹, bevorzugt > 0,5 mm⁻¹, besonders bevorzugt > 1 mm⁻¹. Das Partikelvolumen beträgt < 300 mm³, bevorzugt < 100 mm³.

Die als Formkörper vorliegenden Adsorber werden vorzugsweise in saurem Zustand in ein Festbett eingebracht. Alternativ kann jedoch auch der Einsatz in suspendierter Form oder als Fließ- oder Wirbelbett erfolgen. Der Adsorber kann in einem Zwei- oder Dreiphasencyclus aus Adsorption, optional Spülung und Regenerierung betrieben werden.

Die Fließgeschwindigkeiten liegen bei einer Gasphasenadsorption in einem Festbett bezogen auf den leeren Querschnitt im Bereich von 0,01 bis 2 m/s, bevorzugt 0,1 bis 0,5 m/s. Bei der Flüssigphasenadsorption in einem Festbett liegen die Fließgeschwindigkeiten bezogen auf den leeren Querschnitt im Bereich von 0,01 bis 10 mm/s, bevorzugt im Bereich von 0,1 bis 0,5 mm/s.

Wie beschrieben, erfolgt die Aufarbeitung der wässrigen Salzlösung durch die Entfernung der Base aus der Neutralisation der Säure mit einem Adsorber.

Dabei wird im ersten Schritt, gegebenenfalls nach Einengung, die wässrige Salzlösung über den Adsorber geleitet. Die Adsorbierphase wird beendet, sobald die Konzentration der Base in der austretenden Lösung > 50 %, bevorzugt > 10 %, besonders bevorzugt >1% der eintretenden Lösung ist.

Die wässrige Phase kann durch einen Apparat, insbesondere ein Festbett, in dem sich das Adsorptionsmittel befindet, geleitet werden. Es ist auch möglich, mehrere derartige Apparate hintereinander anzuordnen, oder mehrere derartige Apparate parallel zu schalten, um den Produktstrom bei Bedarf von einem auf den anderen Apparat umzuschalten.

Wenn die Aufnahmekapazität des im Festbett befindlichen Adsorptionsmittels erreicht ist, erfolgt seine Regenerierung. Vor der Regenerierung kann eine Spülphase durchgeführt werden. Dabei wird das im Adsorber bzw. Adsorberbett verbliebene Produkt mit einer Waschflüssigkeit, insbesondere Wasser, ausgespült.

Die Regeneration des Adsorptionsmittels erfolgt durch Desorption der adsorbierten Komponente, beispielsweise durch Temperaturwechsel, Druckwechsel oder Spülen.

Die Regenerierung ist beendet, sobald die Beladung des Adsorbers mit der Base dem Gleichgewichtswert zur Ziel- bzw. Austrittskonzentration der Base während der Adsorptionsphase entspricht.

Im Falle von Ammoniak als Neutralisationsmittel kann die Regenerierung des Adsorptionsmittels auch durch Temperaturerhöhung erfolgen. Diese kann entweder durch direkte Beheizung mit einem erhitzen Fluidstrom oder aber indirekt über Wärmetauscherflächen erfolgen. In einer Desorptionsvariante wird der Adsorber durch überhitztes Gas getrocknet und auf diesem Wege Ammoniak ausgetrieben.

Bei der Verwendung von Ammoniak als Neutralisationsmittel kann die Adsorption auch nach Zersetzung des gebildeten Ammoniumsalzes erfolgen. Dies ist insbesondere bei der Verwendung von Salzsäure als Katalysator und Ammoniak als Neutralisationsmittel in Schritt a) vorteilhaft. Dazu wird die anfallende wässrige Lösung von Ammoniumchlorid verdampft, wobei die Temperatur oberhalb der Zersetzungstemperatur von Ammoniumchlorid in Ammoniak und Chlorwasserstoff liegt. Das gasförmige Gemisch wird dann über den Adsorber geleitet und so die Trennung in Ammoniak und Chlorwasserstoff erreicht. Die Desorption kann durch weitere Temperaturerhöhung, Druckabsenkung oder Spülen erfolgen.

Der Adsorptionsapparat kann in Form eines Festbettes, eine Fließbettes oder eines Wirbelbettes ausgeführt sein. Denkbar ist auch das Suspendieren eines Adsorbers (Rührkessel) mit nachgeschalteter Abscheidung, beispielsweise mittels Filter, Sieb oder Zyklon.

Das erfindungsgemäße Verfahren wird bei der Synthese von Diphenylmethandiamin genutzt. Diese erfolgt in folgenden Schritten:
A) Umsetzung von Anilin mit Formaldehyd in Anwesenheit einer Säure,
B) Neutralisation der Säure mit gasförmigen Ammoniak oder einer ammoniakhaltigen Lösung,
C) Trennung des Reaktionsgemisches aus Schritt B) in eine wässrige und eine organische Phase,
D) Abtrennung der Base aus der in Schritt C) erhaltenen wässrigen Phase durch Behandlung mit einem sauren Adsorber.

Als Säure in Verfahrensschritt A) wird vorzugsweise eine Mineralsäure, insbesondere Salzsäure, eingesetzt.

Die Herstellung des MDA in Schritt A) erfolgt, wie oben beschrieben, durch Umsetzung von Anilin mit Formaldehyd in Gegenwart von Säuren als Katalysatoren. Derartige Verfahren sind allgemein bekannt und beispielsweise im Kunststoffhandbuch, Band 7, Polyurethane, Carl Hanser Verlag München Wien, 3. Auflage, 1993, Seiten 76 bis 86, sowie in einer großen Zahl von Patentanmeldungen, beispielsweise WO 99/40059, beschrieben.

An Stelle von oder im Gemisch mit Formaldehyd kann auch mindestens eine Formaldehyd abspaltende Verbindung eingesetzt werden. Insbesondere wird der Formaldehyd als wässrige Formalinlösung, alkoholische Formalinlösung, Halbacetal, Methylenimin eines primären oder sekundären Amins oder N,N'-Methylendiamin sowie Paraformaldehyd eingesetzt.

Das erfindungsgemäße Verfahren kann kontinuierlich, halbkontinuierlich oder batchweise, vorzugsweise kontinuierlich oder halbkontinuierlich, durchgeführt werden.

Bei der kontinuierlichen Fahrweise werden die Reaktanden in dem gewünschten Verhältnis zueinander in einen Reaktor eindosiert und diesem Reaktor eine dem Zustrom gleiche Menge an Reaktionsprodukt entnommen. Als Reaktoren kommen beispielsweise Rohrreaktoren zum Einsatz. Bei der kontinuierlichen oder halbkontinuierlichen Fahrweise werden die Reaktanden in einen vorzugsweise mit einem Rührer und/oder einem Umpumpkreis versehenen Batchreaktor dosiert, aus dem das ausreagierte Reaktionsprodukt entnommen und der Aufarbeitung zugeführt wird.

Das erfindungsgemäße Verfahren wird vorzugsweise bei einem molaren Verhältnis von Anilin zu Formaldehyd größer 2 durchgeführt. Das molare Verhältnis von Säure zu Anilin ist vorzugsweise größer 0,05. Bei diesen Verhältnissen kommt es zu einer verstärkten Bildung der jeweiligen Zweikernprodukte in der Reaktionsmischung.

Die Reaktion wird vorzugsweise bei einer Temperatur im Bereich zwischen 0 und 200 °C, vorzugsweise zwischen 20 und 150 °C und insbesondere zwischen 40 und 120 °C durchgeführt. Es hat sich gezeigt, dass mit der Erhöhung der Temperatur der Anteil der 2,2'- und 2,4`-Isomeren im Reaktionsprodukt ansteigt.

Der Druck bei der Umsetzung beträgt 0,1 - 50, bevorzugt 1-10 bar absolut.

Bei der batchweisen und halbkontinuierlichen Durchführung der Reaktion kann nach der vollständigen Dosierung der Einsatzstoffe das Reaktionsgemisch einer sogenannten Alterung unterzogen werden. Dazu wird das Reaktionsgemisch im Reaktor belassen oder in einen anderen, vorzugsweise gerührten Reaktor überführt. Dabei liegt die Temperatur des Reaktionsgemisches vorzugsweise über 75 °C, insbesondere in einem Bereich zwischen 110 und 150 °C.

An die Herstellung in Schritt A) schließt sich die Neutralisation b) des Reaktionsgemischs an. Zur Neutralisation können die oben beschriebenen Basen eingesetzt werden.

In einer bevorzugten Ausführungsfrom des Verfahrens wird als Base Ammoniak eingesetzt. Dazu wird der Reaktionsmischung Ammoniak in Gasform oder als wässrige Lösung zugeführt.

Das Gemisch aus Schritt B) liegt in einer organischen und einer anorganischen Phase vor. Diese Phasen werden in Schritt C) getrennt. Die Phasen können beispielsweise durch Dekantieren voneinander getrennt werden. Danach werden die jeweiligen Phasen aufgearbeitet.

Die wässrige Phase, die im wesentlichen aus Wasser, dem darin gelösten Salz der als Katalysator eingesetzten Säure und gegebenenfalls überschüssiger Base, sowie Spuren der Einsatzstoffe Anilin und Formaldehyd und auch des Endprodukts MDA besteht, wird in Schritt D) über einen sauren Adsorber geleitet.

Das in Schritt D) abgetrennte Ammoniak wird gegebenenfalls weiter aufgearbeitet, beispielsweise durch Entfeuchtung, und kann danach wieder in Schritt B) eingesetzt werden.

In Schritt D) wird die Base im Adsorber zurückgehalten, während die als Katalysator eingesetzte Säure den Adsober passiert. Damit enthalten die den Adsorber verlassenen Ströme, die Säuren, die in Schritt A) als Katalysatoren eingesetzt wurden. Diese Säuren können, gegebenenfalls nach vorheriger Aufkonzentration, wieder als Katalysatoren in Schritt A) zurückgeführt beziehungsweise den als Katalysatoren eingesetzten Säuren zugesetzt werden.

Die in Schritt C) abgetrennte organische Phase, die überwiegend aus MDA mit Resten von Wasser, der Base, insbesondere Ammoniak, und den Einsatzprodukten für die Herstellung des MDA besteht, wird ebenfalls aufgearbeitet. Dies erfolgt beispielsweise durch ein- oder mehrmaliges Waschen mit Wasser oder bevorzugt durch mehrfache Destillation zur Abtrennung von beispielsweise Anilin und Wasser.

Durch das erfindungsgemäße Verfahren können auf einfache Weise Mineralsäuren aus großtechnischen Verfahren in hoher Reinheit abgetrennt und zurückgewonnen werden.

Die Erfindung soll an den nachfolgenden Beispielen näher erläutert werden.

### Beispiel 1

500 g eines natürlichen Clinoptilolith-Splits (Material Clinoptilolite, 1-2 mm, Fa. RS Minerals, Guisborough, Cleveland, GB) wurden in eine Austauschersäule vorgelegt. Zur Überführung des Zeoliths in die H-Form wurden 2 I einer 25 %igen wässrigen NH₄Cl-Lösung auf 70 °C temperiert und für 2 Stunden im Kreis über die Austauschersäule gepumpt. Die NH₄Cl-Lösung wurde dann durch 2 I frische 25 %ige wässrige NH₄Cl-Lösung ersetzt und wiederum für 2 Stunden bei 70 °C im Kreis gepumpt. Anschließend wurde mit frischer NH₄Cl-Lösung ein dritter Austauschzyklus in gleicher Weise durchgeführt. Danach wurden 2 I einer 5 %igen wässrigen HCl-Lösung bei Raumtemperatur über die Austauschersäule gepumpt. Abschließend wurde der Zeolith mit Wasser chloridfrei gewaschen, getrocknet (10 Stunden bei 120 °C, Luft) und bei 500 °C im Luftstrom für 5 Stunden calciniert.

Es wurden 426 g des Clinoptiloliths in der H-Form mit einer BET-Oberfläche - ermittelt durch Stickstoffadsorption - von 211 m²/g erhalten. Laut Elementaranalyse weist der Zeolith nach der Behandlung folgende Zusammensetzung auf: 5,7 Gew-% Al, 37 Gew-% Si, 0,09 Gew-% Ca, 0,14 Gew-% Na, 0,38 Gew-% K, 0,85 Gew-% Fe, 0,21 Gew-% Mg.

### Beispiel 2

500 g eines Mordenit-Zeoliths TZM-1013 (Fa. Tricat GmbH, 06749 Bitterfeld, D) wurden mit 25 g Walocel, 178,7 g Methylsilikon Silres^{®} MSE 100 (Fa. Wacker-Chemie, 81737 München, D) und 340 ml Wasser 45 Minuten lang verknetet und anschließend bei einem Pressdruck von 110 bar auf 2 mm Matritzen verstrangt. Die Trocknung erfolgte bei 120 °C an Luft für 6 Stunden, anschließend wurden die Stränge bei 550 °C für 10 Stunden im Luftstrom calciniert.

Es wurden 623 g Zeolithstränglinge mit einem Al-Gehalt von 3,9 Gew.-% erhalten.

### Beispiel 3

65 g des Mordenit-Zeoliths aus Beispiel 2 wurden in einen 250 ml-Glaskolben vorgelegt und mit einer CuCl₂-Lösung, bestehend aus 7 g CuCl₂ und 53,2 g Wasser, versetzt. Die Mischung wurde am Rotationsverdampfer langsam für 30 Minuten bei Raumtemperatur gedreht und anschließend bei 60 °C/35 mbar getrocknet. Die Zeolithstränge wurden dann bei 120 °C an Luft für 12 Stunden getrocknet und abschließend im Luftstrom bei 500 °C für 2 Stunden calciniert.

Es wurden 70,2 g Zeolithstränglinge mit einem Al-Gehalt von 3,9 Gew.-% und einem Cu-Gehalt von 3,1 Gew.-% erhalten.

### Beispiel 4:

In einem Rührbehälter wurden 644,6 g Anilin mit 117,7 g Salzsäure (32 %) versetzt und nach Abkühlen auf unter 60 °C innerhalb von 30 Minuten langsam 201,0 g Formaldehyd (49 %) zudosiert. Die so erhaltene Mischung wurde in einen Druckbehälter überführt und 2 Stunden bei 120 °C unter Eigendruck gerührt. Nach Abkühlen auf unter 60 °C wurde die Mischung mit 60,1 g Ammoniakwasser (30 %) und 500 g Wasser versetzt und die Phasen wurden getrennt, wobei 726 g einer organischen Phase, bestehend überwiegend aus Anilin und MDA, und 796 g wässrige Phase, bestehend überwiegend aus NH₄Cl und Resten von Anilin, erhalten wurden. Die wässrige Phase enthielt: 2,21 g/100g NH₃ und 4,57 g/100g Chlor). Vor Einsatz der wässrigen Lösung in der Adsorption wurden 100 g derselben durch Zugabe von 1200 g Wasser auf eine NH₄Cl-Konzentration von etwa 0,1 mol/l verdünnt. Der Gehalt an NH₃ btrug 0,18 g/100g und der Gehalt an Chlor betrug 0,37 g/100g.

### Beispiel 5:

Die im Beispiel 4 erhaltene wässrige Ammoniumchlorid-Lösung (0,1 mol/l) wurde kontinuierlich bei 300 °C in die Gasphase überführt und über einen bei 300 °C betriebenen Quarzofen geleitet (Belastung: 0,0010 g NH₄Cl /ml*h), der mit 50 ml (34,7 g) Adsorber aus Beispiel 1 befüllt war, wobei der Austrag stündlich analysiert wurde. Zu Beginn wurde eine ammoniakfreie Salzsäurelösung (0,1 mol/l) erhalten. Erst nach einer Laufzeit von 17,5 Stunden war die Kapazität des Adsorbers erschöpft und es befand sich Ammoniak im Austrag. Die Kapazität des Adsorbers betrug also 0,47 mmol NH₃/g. Anschließend wurde der Zulauf abgestellt, die Temperatur im Ofen auf 500 °C erhöht und die gebildeten flüchtigen Bestandteile mit einem Stickstoffstrom ausgestrippt. Nach drei Stunden konnte so der Adsorber wieder regeneriert werden, und der ursprünglich gebundene Ammoniak wurde zurückgewonnen.

### Beispiel 6:

Analog zu Beispiel 5 wurde die wässrige Ammoniumchlorid-Lösung (0,1 mol/l) aus Beispiel 4 bei 300 °C über 50 ml (24,5 g) des in Beispiel 2 beschriebenen Adsorbers geleitet (Belastung 0,0015 g NH₄Cl /ml*h). Nach einer Laufzeit von 7,25 Stunden war die Kapazität des Adsorbers erschöpft und es befand sich Ammoniak im Austrag. Die Kapazität des Adsorbers betrug also 0,41 mmol NH₃/g.

### Beispiel 7:

Analog zu Beispiel 5 wurde die wässrige Ammoniumchlorid-Lösung (0,1 mol/l)) aus Beispiel 4 bei 300 °C über 50 ml (26,2 g) des in Beispiel 3 beschriebenen Adsorbers geleitet (Belastung 0,0011 g NH₄Cl /ml*h). Nach einer Laufzeit von 17,0 Stunden war die Kapazität des Adsorbers erschöpft und es befand sich Ammoniak im Austrag. Die Kapazität des Adsorbers betrug also 0,67 mmol NH₃/g.

## Patentansprüche

1. Verfahren zur Herstellung von Diphenylmethandiamin, umfassend die Schritte
A) Umsetzung von Anilin mit Formaldehyd in Gegenwart einer Mineralsäure,
B) Neutralisation der Mineralsäure mit einer Base,
C) Trennung der organischen und der anorganischen Phase,
D) Entfernung der Base aus der anorganischen Phase mittels Adsorption,
E) Gewinnung der Mineralsäure.

2. Verfahren nach Anspruch 1, **dadurch gekennzeichnet, dass** die Säure ausgewählt ist aus der Gruppe, enthaltend Salzsäure, Schwefelsäure und Salpetersäure.

3. Verfahren nach Anspruch 1, **dadurch gekennzeichnet, dass** die Base ausgewählt ist aus der Gruppe, enthaltend Ammoniak, Hydroxide von Alkalimetallen, primäre, sekundäre oder tertiäre Amine.

4. Verfahren nach Anspruch 1, **dadurch gekennzeichnet, dass** die in Schritt D) verwendeten Adsorber natürliche oder künstliche Silikate sind.

5. Verfahren nach Anspruch 1, **dadurch gekennzeichnet, dass** die in Schritt D) verwendeten Adsorber Zeolithe sind.

6. Verfahren nach Anspruch 1, **dadurch gekennzeichnet, dass** die in Schritt D) adsorbierte Base mittels Desorption wieder entfernt wird.

7. Verfahren nach Anspruch 5 **dadurch gekennzeichnet, dass** als Adsorber Zeolithe mit einer Struktur ausgewählt aus HEU, BEA, MOR, CHA, FAU, MFI, MEL, MWW, MTW, ZBM-11, FER, LTL, MAZ oder MCM-41 eingesetzt werden.

8. Verfahren nach Anspruch 5 **dadurch gekennzeichnet, dass** als Adsorber Zeolithe in der H-Form eingesetzt werden.

9. Verfahren nach Anspruch 5 **dadurch gekennzeichnet, dass** als Adsorber Zeolithe in der H-Form eingesetzt werden, die mit Metallen dotiert sind.

10. Verfahren nach Anspruch 9, **dadurch gekennzeichnet, dass** die dotierten Metalle in einer Menge von 0,5-10 Gew.-%, bezogen auf das Gewicht des Zeoliths, vorhanden sind.

11. Verfahren nach Anspruch 1, **dadurch gekennzeichnet, dass** als Adsorber saure Ionenaustauscher in der H-Form eingesetzt werden.

12. Verfahren nach Anspruch 1, **dadurch gekennzeichnet, dass** die Adsorber als Festbett angeordnet sind.

13. Verfahren nach Anspruch 1, **dadurch gekennzeichnet, dass** die Adsorber als Formkörper eingesetzt werden.

14. Verfahren nach Anspruch 1, **dadurch gekennzeichnet, dass** die Adsorber in Form von Kugeln, Stränglingen, Hohlzylindern, Tabletten, Ringen oder als Split eingesetzt werden.

15. Verfahren nach Anspruch 13, **dadurch gekennzeichnet, dass** das Verhältnis aus äußerer Oberfläche und äußerem Volumen der Formkörper > 0,2 mm⁻¹ beträgt.

## Claims

1. A process for preparing diphenylmethanediamine, which comprises the steps:
A) reaction of aniline with formaldehyde in the presence of a mineral acid,
B) neutralization of the mineral acid with a base,
C) separation of the organic phase and the inorganic phase,
D) removal of the base from the inorganic phase by means of adsorption,
E) recovery of the mineral acid.

2. The process according to claim 1, wherein the acid is selected from the group consisting of hydrochloric acid, sulfuric acid and nitric acid.

3. The process according to claim 1, wherein the base is selected from the group consisting of ammonia, hydroxides of alkali metals, primary, secondary and tertiary amines.

4. The process according to claim 1, wherein the adsorbents used in step D) are natural or synthetic silicates.

5. The process according to claim 1, wherein the adsorbents used in step D) are zeolites.

6. The process according to claim 1, wherein the base adsorbed in step D) is removed again by means of desorption.

7. The process according to claim 5, wherein zeolites having a structure selected from among HEU, BEA, MOR, CHA, FAU, MFI, MEL, MWW, MTW, ZBM-11, FER, LTL, MAZ or MCM-41 are used as adsorbents.

8. The process according to claim 5, wherein zeolites in the H form are used as adsorbents.

9. The process according to claim 5, wherein zeolites which are in the H form and are doped with metals are used as adsorbents.

10. The process according to claim 9, wherein the dopant metals are present in an amount of 0.5-10% by weight, based on the weight of the zeolite.

11. The process according to claim 1, wherein acid ion exchangers in the H form are used as adsorbents.

12. The process according to claim 1, wherein the adsorbents are present as a fixed bed.

13. The process according to claim 1, wherein the adsorbents are used as shaped bodies.

14. The process according to claim 1, wherein the adsorbents are used in the form of spheres, extrudates, hollow cylinders, pellets, rings or crushed material.

15. The process according to claim 13, wherein the ratio of external surface area to external volume of the shaped bodies is > 0.2 mm⁻¹.

## Revendications

1. Procédé pour la préparation de diphénylméthanediamine, comprenant les étapes
A) transformation d'aniline avec du formaldéhyde en présence d'un acide minéral,
B) neutralisation de l'acide minéral avec une base,
C) séparation de la phase organique et inorganique,
D) élimination de la base de la phase inorganique par adsorption,
E) extraction de l'acide minéral.

2. Procédé selon la revendication 1, **caractérisé en ce que** l'acide est choisi dans le groupe contenant l'acide chlorhydrique, l'acide sulfurique et l'acide nitrique.

3. Procédé selon la revendication 1, **caractérisé en ce que** la base est choisie dans le groupe contenant l'ammoniac, les hydroxydes de métaux alcalins, les amines primaires, secondaires ou tertiaires.

4. Procédé selon la revendication 1, **caractérisé en ce que** les adsorbants utilisés dans l'étape D) sont des silicates naturels ou synthétiques.

5. Procédé selon la revendication 1, **caractérisé en ce que** les adsorbants utilisés dans l'étape D) sont des zéolithes.

6. Procédé selon la revendication 1, **caractérisé en ce que** la base adsorbée dans l'étape D) est à nouveau éliminée par désorption.

7. Procédé selon la revendication 5, **caractérisé en ce qu'**on utilise, comme adsorbants, des zéolithes présentant une structure choisie parmi HEU, BEA, MOR, CHA, FAU, MFI, MEL, MWW, MTW, ZBM-11, FER, LTL, MAZ ou MCM-41.

8. Procédé selon la revendication 5, **caractérisé en ce qu'**on utilise comme adsorbants des zéolithes sous forme H.

9. Procédé selon la revendication 5, **caractérisé en ce qu'**on utilise comme adsorbants des zéolithes sous forme H, qui sont dopées avec des métaux.

10. Procédé selon la revendication 9, **caractérisé en ce que** les métaux de dopage sont présents en une quantité de 0,5-10% en poids, par rapport au poids de la zéolithe.

11. Procédé selon la revendication 1, **caractérisé en ce qu'**on utilise comme adsorbants des échangeurs d'ions acides sous forme H.

12. Procédé selon la revendication 1, **caractérisé en ce que** les adsorbants sont disposés sous forme de lit fixe.

13. Procédé selon la revendication 1, **caractérisé en ce que** les adsorbants sont utilisés sous forme de corps moulé.

14. Procédé selon la revendication 1, **caractérisé en ce que** les adsorbants sont utilisés sous forme de billes, de brins extrudés, de cylindres creux, de comprimés, d'anneaux ou de fragments.

15. Procédé selon la revendication 13, **caractérisé en ce que** le rapport de la surface extérieure au volume extérieur des corps moulés est > 0,2 mm⁻¹.
